# EUROPEAN PATENT APPLICATION

(11) **EP 4 725 951 A1**
(43) Date of publication of application: **15.04.2026**
(21) Application number: 24842304.8
(22) Date of filing: 12.07.2024
(51) Int. Cl.: C07J 63/00, A61K 31/57, C07J 61/00, A61P 25/00

(54) **CRYSTAL FORM OF STEROID COMPOUND AND USE THEREOF**

(30) Priority: 14.07.2023 CN 202310871702
(71) Applicant: Chengdu Kanghong Pharmaceutical Co., Ltd., Chengdu, Sichuan 610036 (CN)
(72) Inventor: KE, Zunhong, Chengdu, Sichuan 610036 (CN); CHEN, Pei, Chengdu, Sichuan 610036 (CN); CHEN, Minglong, Chengdu, Sichuan 610036 (CN); ZHANG, Jun, Chengdu, Sichuan 610036 (CN); XIONG, Xinnuo, Chengdu, Sichuan 610036 (CN)
(74) Representative: WSL Patentanwälte Partnerschaft mbB
(86) International application number: PCT/CN2024/105193
(87) International publication number: WO 2025/016311

(57) **Abstract**

The purpose of the present invention is to provide a crystal form of a steroid compound, a pharmaceutical composition and the use thereof in the preparation of a drug for diseases related to a GABAA receptor. Compared with the prior art, the crystal form provided in the present invention has one or more improved properties, and has great significance in further development of the drug.

## Description

### TECHNICAL FIELD

The present invention relates to the field of chemical pharmaceuticals, and specifically to crystal form of steroid compound and use thereof.

### BACKGROUND

Neuropsychiatric disorders, including anxiety disorders, depression, schizophrenia and others, rank first in the total disease burden in China, accounting for approximately 20% of the total disease burden. With the continuous development of social modernization, the accelerated pace of work, and increased life pressure, the number of patients with various neuropsychiatric disorders has increased significantly, and the progression of symptoms has accelerated markedly. This makes the development, research and production of drugs for treating neuropsychiatric disorders even more urgent.

The GABA(A) receptor (GABAAR) is an ionic receptor and ligand-gated ion channel. Its endogenous ligand, γ-aminobutyric acid (GABA), acts as an inhibitory neurotransmitter in the central nervous system. The GABA system is the primary inhibitory signaling pathway in the brain and central nervous system, playing a crucial role in regulating central nervous system functions. Upon activation, the GABA(A) receptor selectively allows chloride ions (Cl⁻) to pass through its pore. Cl⁻ flows out of the neuron when the internal voltage is less than the resting potential and flows into the neuron when the internal voltage is greater than the resting potential (i.e., -75 mV). This successfully reduces the likelihood of action potential generation, thereby exerting an inhibitory effect on neurotransmission. Research findings confirm that the GABAergic system and GABA(A) receptors play significant roles in the etiology and pathology of diseases such as anxiety, depression, and schizophrenia. The GABAergic system and GABA(A) receptors have been demonstrated to be involved in the pathological processes of anxiety, depression, and schizophrenia at the molecular, preclinical, and clinical levels. Furthermore, the GABA(A) receptor has long been recognized as an important drug target for treating these diseases.

Many studies are based on the GABA(A) receptor, aiming to obtain drugs that can effectively treat related diseases. For example, WO2020143640A1 provides a class of steroid compounds that modulate brain excitability by regulating GABA(A) receptor for the treatment of neuropsychiatric disorders.

Polymorphism of drugs refers to the different lattice arrangements formed by the ordered arrangement of chemical drug molecules in their microstructure, generally manifested as different solid-state forms of the drug substance. A single drug can exist in multiple crystalline forms. Different crystal forms of the same drug may exhibit different physical or chemical properties. Research on drug polymorphism is of great significance for developing crystal forms more suitable for pharmaceutical use.

### SUMMARY OF THE INVENTION

An object of the present invention is to provide a new crystal form of the compound of Formula I as described below, designated as crystal form A.

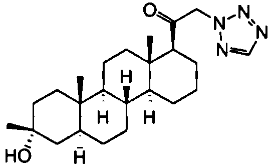

In some embodiments, the crystal form A provided by the present invention is characterized in that the X-ray powder diffraction pattern thereof has characteristic peaks at 2θ values of 8.1°±0.2°, 11.7°±0.2°, 13.5°±0.2°, 16.2°±0.2°, and 17.6°±0.2°.

In some embodiments, the crystal form A provided by the present invention is characterized in that the X-ray powder diffraction pattern thereof has characteristic peaks at 2θ values of 8.1°±0.2°, 11.7°±0.2°, 13.0°±0.2°, 13.5°±0.2°, 15.3°±0.2°, 16.2°±0.2°, 17.6°±0.2°, and 21.3°±0.2°.

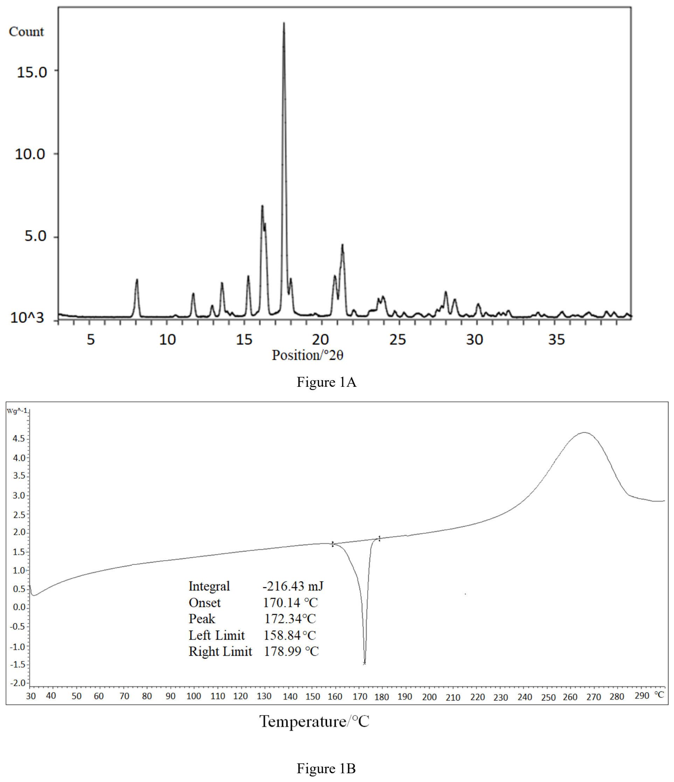

In some embodiments, the crystal form A provided by the present invention is characterized in that the X-ray powder diffraction pattern thereof is as shown in Figure 1A.

In some embodiments, the present invention provides a method for preparing the crystal form A, which comprises adding the compound of Formula I into an organic solvent to achieve complete dissolution, then lowering the temperature to induce precipitation, and obtaining a solid after centrifugation. In some specific embodiments, the organic solvent is isopropanol, ethyl propyl acetate, or acetonitrile, preferably isopropanol. In other specific embodiments, the organic solvent is a mixed solvent of isopropanol and water, ethanol and water, or acetone and water; wherein the volume ratio of isopropanol to water, ethanol to water, or acetone to water is 1:1 to 1:5, preferably 1:1 to 1:2. In yet other specific embodiments, the organic solvent is a mixed solvent of acetonitrile and 2-methyltetrahydrofuran, wherein the volume ratio of acetonitrile to 2-methyltetrahydrofuran is 1:1 to 1:5, preferably 1:1 to 1:2. In some preferred embodiments, the mass-to-volume ratio of the compound of Formula I to the organic solvent is 100:1 to 5:1 (mg:mL), preferably 50:1 to 10:1, more preferably 20:1.

In other embodiments, the method for preparing the crystal form A provided by the present invention can also be carried out using conventional methods in the field, such as slow evaporation, low-temperature (4-8°C) slurry conversion, room-temperature slurry conversion, high-temperature (50°C) slurry conversion, anti-solvent crystallization, gas-solid diffusion, gas-liquid diffusion, vapor-simulated crystallizaton, polymer induction, grinding, cyclic heating and cooling, rotary evaporation, and the like. For example, in the low-temperature (4-8°C) slurry conversion method, an organic solvent such as isopropanol, isopropyl acetate, methyl tert-butyl ether, or acetonitrile is added to the compound of Formula I. The resulting suspension is stirred magnetically (for instance, for 3 days, 4 days, 5 days, or even more than 6 days) at low temperature (e.g., 5°C), followed by centrifugation to collect the solid. For example, in the anti-solvent addition method, the good solvent can be selected from the group consisting of acetone, tetrahydrofuran, 1,4-dioxane, acetonitrile, acetone, 2-methyltetrahydrofuran, isopropanol, dichloromethane, and methyl tert-butyl ether, etc., and the anti-solvent is selected from the group consisting of water and n-heptane, etc. For example, in the rotary evaporation method, the solvent can be selected from the group consisting of acetone and tetrahydrofuran, etc.

Another object of the present invention is to provide a new crystal form of the compound of Formula I, designated as crystal form B.

In some embodiments, the crystal form B provided by the present invention is characterized in that the X-ray powder diffraction pattern thereof has characteristic peaks at 2θ values of 7.3°+0.2°, 9.0°±0.2°, 14.7°±0.2°, 15.6°±0.2°, and 18.0°+0.2°.

In some embodiments, the crystal form B provided by the present invention is characterized in that the X-ray powder diffraction pattern thereof has characteristic peaks at 2θ values of 7.3°+0.2°, 9.0°±0.2°, 11.0°±0.2°, 12.2°±0.2°, 14.7°±0.2°, 15.3°±0.2°, 15.6°+0.2°, and 18.0°+0.2°.

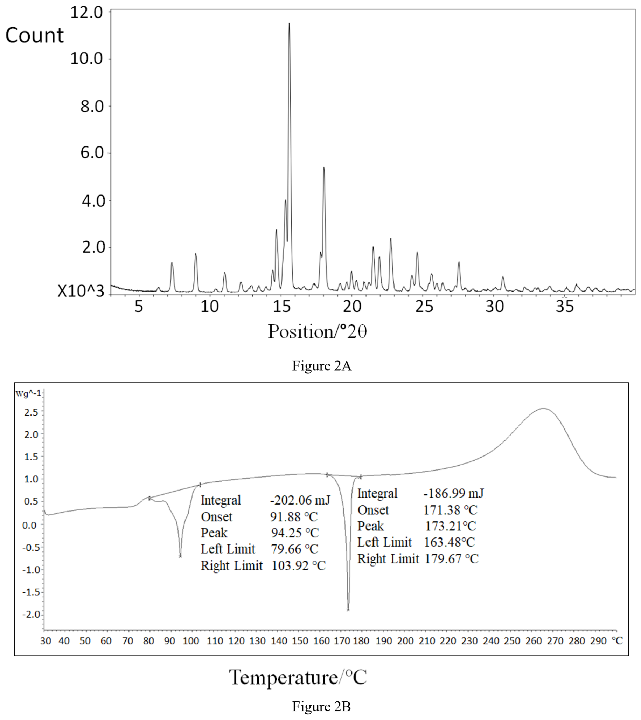

In some embodiments, the crystal form B provided by the present invention is characterized in that the X-ray powder diffraction pattern thereof is as shown in Figure 2A.

In some embodiments, the present invention provides a method for preparing the crystal form B. The preparation method comprises adding the compound of Formula I to a mixed solvent of isopropyl acetate and toluene, or a mixed solvent of ethanol and toluene, followed by suspension stirring (e.g., for 3 days, 4 days, 5 days, or even more than 6 days) at a low temperature (e.g., 5°C) or room temperature to obtain the crystal form B.

In other embodiments, crystal form transformation experiments were conducted on crystal form B. It was found that after crystal form B was left uncovered at 50°C for 5 hours and analyzed by XRPD, it transformed into crystal form A. In another transformation experiment, when crystal form B was heated to 110°C by DSC and held at this temperature for 3 minutes before cooling to room temperature, subsequent XRPD analysis revealed that crystal form B had transformed into a mixture of crystal form A and crystal form D.

Another object of the present invention is to provide a new crystal form of the compound of Formula I, designated as crystal form C.

In some embodiments, the crystal form C provided by the present invention is characterized in that the X-ray powder diffraction pattern thereof has characteristic peaks at 2θ values of 5.6°+0.2°, 8.6°±0.2°, 14.0°±0.2°, 16.7°±0.2°, and 17.4°±0.2°.

In some embodiments, the crystal form C provided by the present invention is characterized in that the X-ray powder diffraction pattern thereof has characteristic peaks at 2θ values of 5.6°±0.2°, 8.6°±0.2°, 9.9°±0.2°, 14.0°±0.2°, 14.9°±0.2°, 16.7°±0.2°, 17.4°±0.2°, and 18.5°±0.2°.

In some embodiments, the crystal form C provided by the present invention is characterized in that the X-ray powder diffraction pattern thereof has characteristic peaks at 2θ values of 5.6°±0.2°, 8.6°±0.2°, 9.9°±0.2°, 11.2°±0.2°, 11.8°±0.2°, 14.0°±0.2°, 14.9°±0.2°, 16.7°±0.2°, 17.4°±0.2°, 18.5°±0.2°, and 18.9°±0.2°.

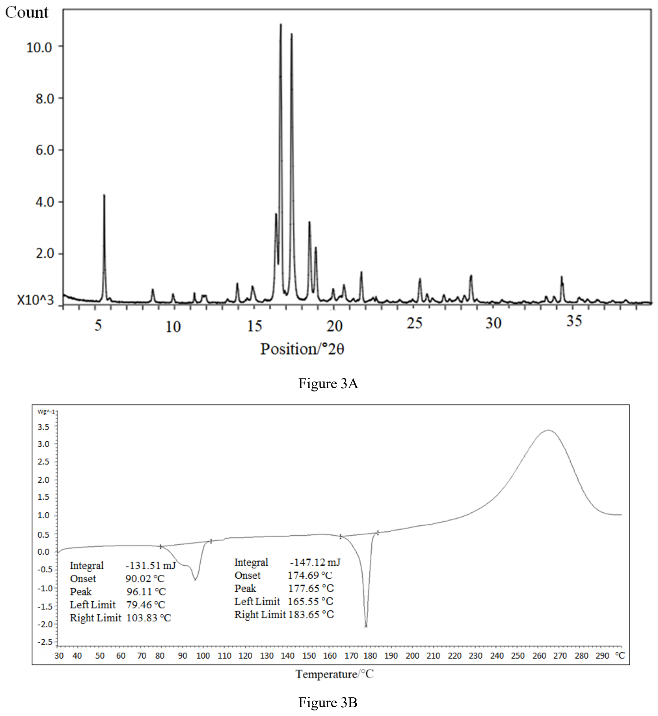

In some embodiments, the crystal form C provided by the present invention is characterized in that the X-ray powder diffraction pattern thereof is as shown in Figure 3A.

In some embodiments, the present invention provides a method for preparing the crystal form C. The preparation method comprises adding the compound of Formula I to methanol, subjecting the mixture to sonication to promote dissolution, filtering, and allowing the filtrate to stand and evaporate to obtain the crystal form C.

In a crystal form transformation experiment, crystal form C was left uncovered at 50°C for approximately 4 hours. The XRPD analysis showed that the crystal form remained unchanged. Crystal form C was heated to 110°C by DSC and held at this temperature for 3 minutes before cooling to room temperature. XRPD analysis revealed that crystal form C had transformed into crystal form G.

Another object of the present invention is to provide a new crystal form of the compound of Formula I, designated as crystal form D.

In some embodiments, the crystal form D provided by the present invention is characterized in that the X-ray powder diffraction pattern thereof has characteristic peaks at 2θ values of 7.1°+0.2°, 8.8°±0.2°, 12.2°±0.2°, 14.4°±0.2°, and 17.6°±0.2°.

In some embodiments, the crystal form D provided by the present invention is characterized in that the X-ray powder diffraction pattern thereof has characteristic peaks at 2θ values of 7.1°±0.2°, 8.8°±0.2°, 10.2°±0.2°, 12.2°±0.2°, 12.9°±0.2°, 13.5°±0.2°, 14.4°±0.2°, and 17.6°±0.2°.

In some embodiments, the crystal form D provided by the present invention is characterized in that the X-ray powder diffraction pattern thereof has characteristic peaks at 2θ values of 7.1°+0.2°, 7.6°±0.2°, 8.0°±0.2°, 8.8°+0.2°, 10.2°±0.2°, 11.0°±0.2°, 11.6°±0.2°, 12.2°±0.2°, 12.9°+0.2°, 13.5°±0.2°, 14.0°±0.2°, 14.4°±0.2°, 15.4°±0.2°, 17.6°±0.2°, and 18.2°±0.2°.

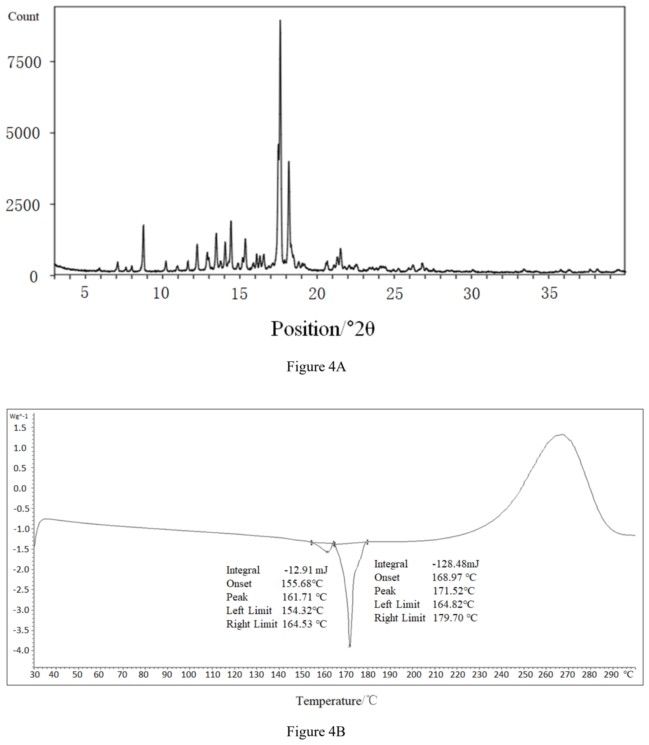

In some embodiments, the crystal form D provided by the present invention is characterized in that the X-ray powder diffraction pattern thereof is as shown in Figure 4A.

In some embodiments, the present invention provides a method for preparing the crystal form D. The preparation method comprises adding the compound of Formula I to dichloromethane, subjecting the mixture to sonication to promote dissolution, filtering, and obtaining the crystal form D by rotary evaporation.

In a crystal form transformation experiment, crystal form D was heated to 160°C and held at 160°C for 3 minutes before cooling to room temperature. XRPD analysis revealed that crystal form D had transformed into a mixture of crystal form A and crystal form G after the high-temperature treatment.

Another object of the present invention is to provide a new crystal form of the compound of Formula I, designated as crystal form E.

In some embodiments, the crystal form E provided by the present invention is characterized in that the X-ray powder diffraction pattern thereof has characteristic peaks at 2θ values of 6.1°±0.2°, 12.4°±0.2°, 13.8°+0.2°, 15.8°+0.2°, and 17.9°±0.2°.

In some embodiments, the crystal form E provided by the present invention is characterized in that the X-ray powder diffraction pattern thereof has characteristic peaks at 2θ values of 6.1°±0.2°, 12.4°±0.2°, 13.8°±0.2°, 14.6°±0.2°, 15.2°±0.2°, 15.8°±0.2°, 17.9°±0.2°, and 18.7°±0.2°.

In some embodiments, the crystal form E provided by the present invention is characterized in that the X-ray powder diffraction pattern thereof has characteristic peaks at 2θ values of 6.1°±0.2°, 10.1°±0.2°, 12.4°±0.2°, 13.8°±0.2°, 14.6°±0.2°, 15.2°±0.2°, 15.8°+0.2°, 17.6°±0.2°, 17.9°±0.2°, 18.7°±0.2°, 20.0°±0.2°, 21.6°±0.2°, and 22.9°±0.2°.

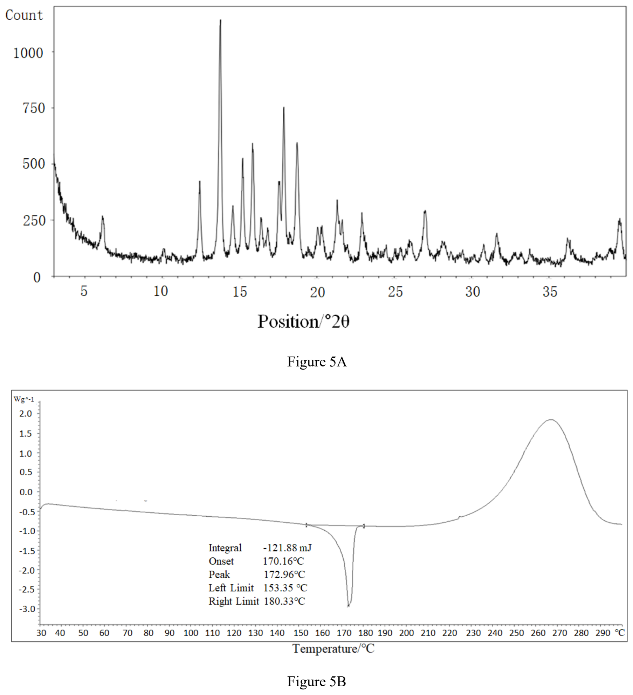

In some embodiments, the crystal form E provided by the present invention is characterized in that the X-ray powder diffraction pattern thereof is as shown in Figure 5A.

In some embodiments, the present invention provides a method for preparing crystal form E. The preparation method comprises adding the compound of Formula I to methanol, subjecting the mixture to sonication to promote dissolution, filtering to obtain a filtrate, slowly dripping the filtrate into a vial pre-filled with water, yielding crystal form E upon solid precipitation.

In another embodiment, the present invention provides a method for preparing crystal form E. The preparation method comprises adding the compound of Formula I to methanol, subjecting the mixture to sonication to promote dissolution, filtering to obtain a filtrate, then, adding the filtrate to a container containing a certain volume of purified water to precipitate solid, stirring the mixture at room temperature, filtering to collect the solid, and drying the solid in an oven at a high temperature (e.g., 50°C) overnight.

Another object of the present invention is to provide a new crystal form of the compound of Formula I, designated as crystal form F.

In some embodiments, the crystal form F provided by the present invention is characterized in that the X-ray powder diffraction pattern thereof has characteristic peaks at 2θ values of 8.7°+0.2°, 14.9°+0.2°, 16.4°±0.2°, 17.5°±0.2°, and 20.4°±0.2°.

In some embodiments, the crystal form F provided by the present invention is characterized in that the X-ray powder diffraction pattern thereof has characteristic peaks at 2θ values of 8.7°±0.2°, 12.0°+0.2°, 14.6°±0.2°, 14.9°±0.2°, 16.4°±0.2°, 17.5°±0.2°, 18.7°±0.2°, and 20.4°±0.2°.

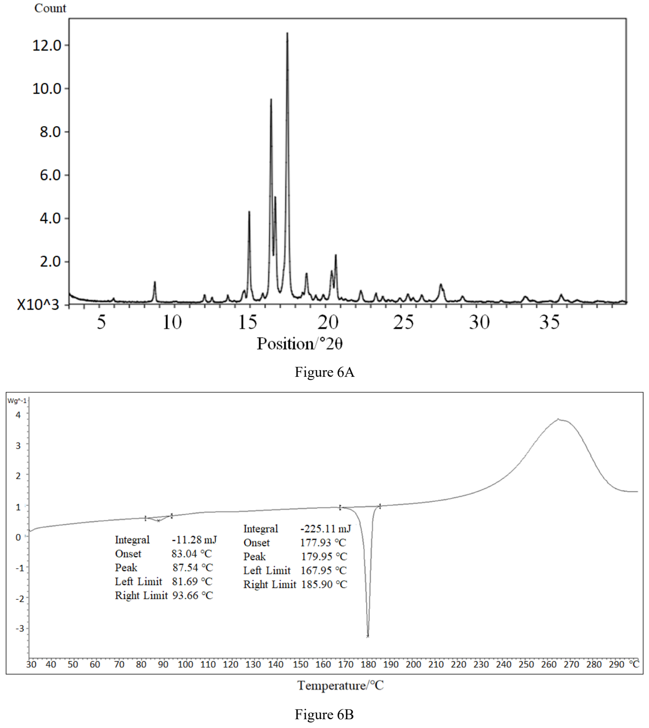

In some embodiments, the crystal form F provided by the present invention is characterized in that the X-ray powder diffraction pattern thereof is as shown in Figure 6A.

In some embodiments, the present invention provides a method for preparing crystal form F. The preparation method comprises adding the compound of Formula I to methanol, the mixture was stirred at room temperature overnight (for approximately 18 hours), filtering, and drying the solid at 50°C overnight (for approximately 18 hours) to obtain the crystal form F.

Another object of the present invention is to provide a new crystal form of the compound of Formula I, designated as crystal form G.

In some embodiments, the crystal form G provided by the present invention is characterized in that the X-ray powder diffraction pattern thereof has characteristic peaks at 2θ values of 8.4°±0.2°, 10.1°±0.2°, 13.7°±0.2°, 16.8°±0.2°, and 17.6°±0.2°.

In some embodiments, the crystal form G provided by the present invention is characterized in that the X-ray powder diffraction pattern thereof has characteristic peaks at 2θ values of 8.4°+0.2°, 10.1°±0.2°, 13.7°±0.2°, 14.6°±0.2°, 15.2°±0.2°, 15.8°±0.2°, 16.8°±0.2°, and 17.6°±0.2°.

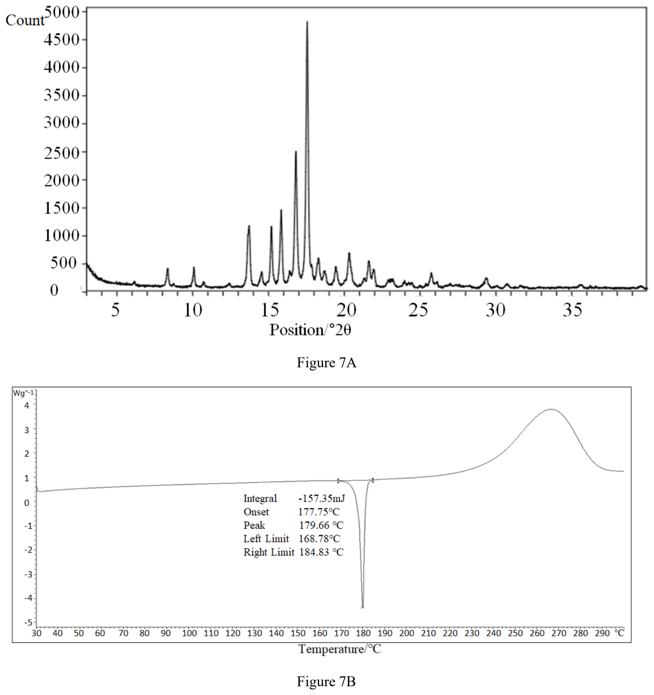

In some embodiments, the crystal form G provided by the present invention is characterized in that the X-ray powder diffraction pattern thereof is as shown in Figure 7A.

In some embodiments, the present invention provides a method for preparing the crystal form G. The preparation method comprises placing crystal form F of the compound of Formula I on a hot stage, heating to 125°C and maintaining for approximately 10 minutes to transform it into crystal form G.

In some embodiments, the present invention provides a pharmaceutical composition comprising any one of crystal form A, crystal form B, crystal form C, crystal form D, crystal form E, crystal form F, or crystal form G of the compound of Formula I according to the present invention, and a pharmaceutically acceptable excipient.

In some embodiments, the present invention provides the use of any one of crystal form A, crystal form B, crystal form C, crystal form D, crystal form E, crystal form F, or crystal form G of the compound of Formula I according to the present invention in the preparation of a medicament for a disease related to a GABAA receptor.

In some embodiments, the disease related to the GABAA receptor according to the present invention is a nervous system disease or a metabolic disease.

In some embodiments, the nervous system disease according to the present invention is selected from the group consisting of: sleep disorders, mood disorders, schizophrenia spectrum disorders, spastic disorders, memory disorders and/or cognitive disorders, movement disorders, personality disorders, autism spectrum disorders, pain, traumatic brain injury, vascular diseases, substance abuse disorders and/or withdrawal syndromes, and tinnitus.

In some preferred embodiments, the mood disorder is depression; wherein the depression comprises disruptive mood dysregulation disorder, major depressive disorder, persistent depressive disorder, premenstrual dysphoric disorder, substance/medication-induced disorder, disorders due to other physical disease, other specified depressive disorder and unspecified depressive disorder.

In some preferred embodiments, the depression is selected from the group consisting of mild depression, moderate depression, major depression, and postpartum depression.

In some preferred embodiments, the nervous system disease is major depression or postpartum depression.

In some embodiments, the metabolic disease is selected from the group consisting of obesity, hyperlipidemia and hypertension, etc.

The advantageous effects of the present invention are as follows: The present invention provides a series of crystal forms of the compound of Formula I suitable for pharmaceutical use. These crystal forms are free of solvate compounds or solvent residues and exhibit good thermal stability. In other preferred crystal forms, the crystal forms of the present invention also possess good storage stability (e.g., resistance to high temperature, high humidity, and light exposure) and/or grinding stability, making them well-suited for industrial-scale production and application.

### DESCRIPTION OF THE DRAWINGS

Figure 1A is the X-ray powder diffraction pattern of crystal form A according to Example 1 of the present invention;
Figure 1B is the DSC curve of crystal form A according to Example 1 of the present invention;
Figure 1C is the TGA curve of crystal form A according to Example 1 of the present invention;
Figure 1D is the ¹H NMR spectrum of crystal form A according to Example 1 of the present invention;
Figure 2A is the X-ray powder diffraction pattern of crystal form B according to Example 2 of the present invention;
Figure 2B is the DSC curve of crystal form B according to Example 2 of the present invention;
Figure 2C is the ¹H NMR spectrum of crystal form B according to Example 2 of the present invention;
Figure 3A is the X-ray powder diffraction pattern of crystal form C according to Example 3 of the present invention;
Figure 3B is the DSC curve of crystal form C according to Example 3 of the present invention;
Figure 3C is the ¹H NMR spectrum of crystal form C according to Example 3 of the present invention;
Figure 4A is the X-ray powder diffraction pattern of crystal form D according to Example 4 of the present invention;
Figure 4B is the DSC curve of crystal form D according to Example 4 of the present invention;
Figure 4C is the ¹H NMR spectrum of crystal form D according to Example 4 of the present invention;
Figure 5A is the X-ray powder diffraction pattern of crystal form E according to Example 5 of the present invention;
Figure 5B is the DSC curve of crystal form E according to Example 5 of the present invention;
Figure 5C is the ¹H NMR spectrum of crystal form E according to Example 5 of the present invention;
Figure 6A is the X-ray powder diffraction pattern of crystal form F according to Example 6 of the present invention;
Figure 6B is the DSC curve of crystal form F according to Example 6 of the present invention;
Figure 6C is the ¹H NMR spectrum of crystal form F according to Example 6 of the present invention;
Figure 7A is the X-ray powder diffraction pattern of crystal form G according to Example 7 of the present invention;
Figure 7B is the DSC curve of crystal form G according to Example 7 of the present invention;
Figure 7C is the ¹H NMR spectrum of crystal form G according to Example 7 of the present invention;
Figure 8A is the X-ray powder diffraction pattern of crystal form H according to Example 8 of the present invention;
Figure 8B is the DSC curve of crystal form H according to Example 8 of the present invention;
Figure 9A is the X-ray powder diffraction pattern of crystal form A according to Example 10 of the present invention;
Figure 9B is the X-ray powder diffraction pattern of crystal form G according to Example 10 of the present invention.

### DETAILED DESCRIPTION

The present invention is further described in detail through the following examples, which are not intended to limit the scope of protection of the present invention.

### Testing Instruments and Methods:

X-ray Powder Diffraction (XRPD) testing method: Instrument model: D8 Advance, radiation source: CuK (40 kV, 40 mA), step size: 0.02° 2θ, scanning speed: 0.1 s/step, scanning range: 3° to 40° (2θ).

Differential Scanning Calorimetry (DSC) testing method: Instrument model: DSC 3; parameters: Nitrogen (N₂) protection, gas flow rate: 50 mL/min, heating rate: 10°C/min, temperature range: 30°C to 300°C.

Thermogravimetric Analysis (TGA): Instrument model: TGA 2; parameters: Nitrogen (N₂) protection, gas flow rate: 50 mL/min, heating rate: 10.0 k/min, temperature range: 30°C to 350°C.

¹H NMR: Instrument model: Bruker AVANCE III 400 MHz; parameters: Full-frequency excitation, spectral width: 20 ppm, single pulse, 30° excitation scanning for 8 times, digital quadrature detection, temperature control at 298 K; solvent: DMSO-d₆.

The compound of Formula I ((1-((1S,4aS,4bR,6aS,8R,10aS,10bS,12aS)-8-hydroxy-8,10a,12a-trimethyloctadecahydrochrysen-1-yl)-2-(2H-tetrazol-2-yl)ethan-1-one) can be obtained commercially or self-prepared. For example, it can be prepared according to the method described in Example 20 of the prior art patent CN202010015181.0 (WO2020143640A1).

### Example 1: Preparation of crystal form A

Approximately 20 mg of the compound of Formula I was added to a 3 mL vial. 1 mL of isopropanol was added, and the mixture was sonicated at 50°C to promote dissolution. The solution was then filtered through a 0.45 µm PTFE filter into another 3 mL vial. This clear filtrate was stirred at 50°C and allowed to cool naturally to room temperature, resulting in solid precipitation. The precipitated solid was collected by centrifugation and subjected to XRPD testing.

The X-ray powder diffraction pattern is as shown in Figure 1A. The XRPD data are listed in Table 1.

**Table 1**

| Diffraction Angle [°2θ] | d value [Å] | Peak Height | I% | Area | I% |
|---|---|---|---|---|---|
| 8.083 | 10.9288 | 2267 | 12.9 | 28356 | 12.8 |
| 10.541 | 8.3857 | 126 | 0.7 | 1481 | 0.7 |
| 11.708 | 7.5523 | 1416 | 8.1 | 15322 | 6.9 |
| 12.951 | 6.8299 | 651 | 3.7 | 6582 | 3 |
| 13.541 | 6.5335 | 2029 | 11.6 | 28762 | 13 |
| 14.203 | 6.2305 | 235 | 1.3 | 2639 | 1.2 |
| 15.253 | 5.8039 | 2380 | 13.6 | 25214 | 11.4 |
| 16.168 | 5.4774 | 6577 | 37.4 | 99576 | 44.9 |
| 16.343 | 5.4192 | 5495 | 31.3 | 152049 | 68.5 |
| 17.552 | 5.0487 | 17563 | 100 | 221941 | 100 |
| 17.981 | 4.929 | 2160 | 12.3 | 40599 | 18.3 |
| 19.525 | 4.5428 | 156 | 0.9 | 2013 | 0.9 |
| 20.826 | 4.2618 | 2430 | 13.8 | 41404 | 18.7 |
| 21.33 | 4.1621 | 4288 | 24.4 | 78503 | 35.4 |
| 22.07 | 4.0242 | 362 | 2.1 | 3709 | 1.7 |
| 23.183 | 3.8335 | 373 | 2.1 | 18282 | 8.2 |
| 23.633 | 3.7616 | 1055 | 6 | 32533 | 14.7 |
| 23.923 | 3.7166 | 1183 | 6.7 | 35658 | 16.1 |
| 24.684 | 3.6037 | 331 | 1.9 | 3071 | 1.4 |
| 25.287 | 3.5191 | 283 | 1.6 | 2721 | 1.2 |
| 26.161 | 3.4035 | 209 | 1.2 | 4712 | 2.1 |
| 26.882 | 3.3138 | 170 | 1 | 1882 | 0.8 |
| 27.429 | 3.249 | 384 | 2.2 | 6328 | 2.9 |
| 27.975 | 3.1868 | 1471 | 8.4 | 35795 | 16.1 |
| 28.559 | 3.1229 | 893 | 5.1 | 11875 | 5.4 |
| 29.283 | 3.0474 | 114 | 0.6 | 956 | 0.4 |
| 30.078 | 2.9686 | 744 | 4.2 | 10362 | 4.7 |
| 30.584 | 2.9206 | 239 | 1.4 | 3126 | 1.4 |
| 31.42 | 2.8448 | 247 | 1.4 | 2900 | 1.3 |
| 31.693 | 2.8209 | 236 | 1.3 | 4235 | 1.9 |
| 32.008 | 2.7938 | 376 | 2.1 | 4821 | 2.2 |
| 33.604 | 2.6647 | 131 | 0.7 | 3481 | 1.6 |
| 33.917 | 2.6408 | 297 | 1.7 | 6526 | 2.9 |
| 34.326 | 2.6103 | 171 | 1 | 2830 | 1.3 |
| 35.529 | 2.5246 | 337 | 1.9 | 5127 | 2.3 |
| 36.156 | 2.4822 | 116 | 0.7 | 2293 | 1 |
| 36.451 | 2.4628 | 110 | 0.6 | 2326 | 1 |
| 37.208 | 2.4145 | 295 | 1.7 | 6161 | 2.8 |
| 38.357 | 2.3447 | 346 | 2 | 4769 | 2.1 |
| 38.827 | 2.3175 | 302 | 1.7 | 4178 | 1.9 |

The DSC curve is as shown in Figure 1B, displaying an endothermic peak at approximately 170.14°C (onset value).

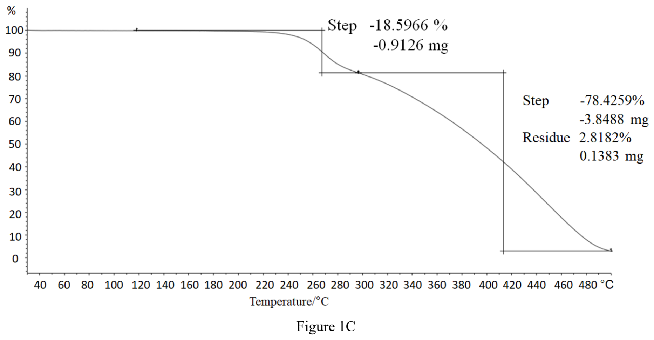

The TGA curve is as shown in Figure 1C. Crystal form A exhibited no weight loss before 120°C, indicating it is an anhydrous crystal form.

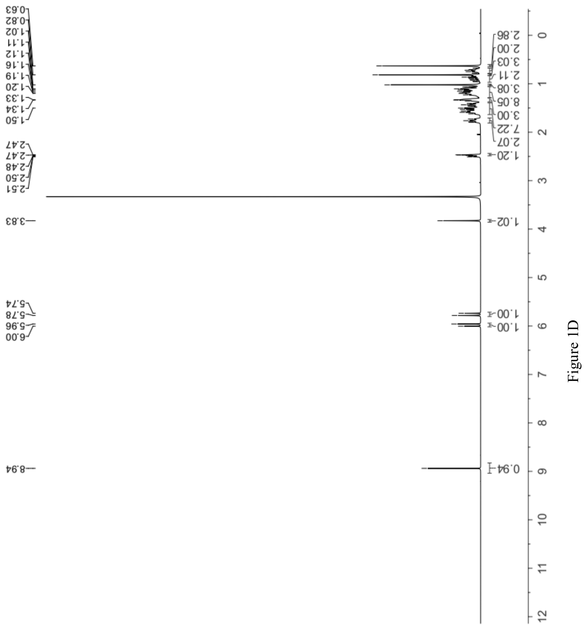

The ¹H NMR spectrum is as shown in Figure 1D. The ¹H NMR analysis indicated the absence of residual solvent.

### Example 2: Preparation of crystal form B

Approximately 30 mg of the compound of Formula I was added to a 3 mL vial. Then, 0.5 mL of a mixed solvent of isopropyl acetate and toluene (1:4) was added to the vial. A stir bar was added, and the suspension was stirred at 5°C for 5 days.

The X-ray powder diffraction pattern is as shown in Figure 2A. The XRPD data are listed in Table 2.

**Table 2**

| Diffraction Angle [°2θ] | d value [Å] | Peak Height | I% | Area | I% |
|---|---|---|---|---|---|
| 6.389 | 13.8233 | 175 | 1.6 | 1844 | 1.5 |
| 7.305 | 12.0916 | 1242 | 11 | 14298 | 11.7 |
| 8.981 | 9.838 | 1639 | 14.6 | 18119 | 14.8 |
| 10.367 | 8.5261 | 121 | 1.1 | 1123 | 0.9 |
| 11.009 | 8.03 | 834 | 7.4 | 8879 | 7.2 |
| 12.158 | 7.2735 | 414 | 3.7 | 4280 | 3.5 |
| 12.935 | 6.8386 | 248 | 2.2 | 3552 | 2.9 |
| 13.442 | 6.5818 | 245 | 2.2 | 2145 | 1.7 |
| 13.981 | 6.3293 | 153 | 1.4 | 855 | 0.7 |
| 14.417 | 6.1388 | 907 | 8.1 | 13378 | 10.9 |
| 14.687 | 6.0264 | 2625 | 23.3 | 29881 | 24.3 |
| 15.331 | 5.7748 | 3732 | 33.1 | 73554 | 59.9 |
| 15.585 | 5.681 | 11264 | 100 | 122726 | 100 |
| 16.233 | 5.4557 | 53 | 0.5 | 246 | 0.2 |
| 16.636 | 5.3246 | 138 | 1.2 | 1130 | 0.9 |
| 17.356 | 5.1052 | 272 | 2.4 | 3735 | 3 |
| 17.805 | 4.9774 | 1627 | 14.4 | 53326 | 43.5 |
| 18.022 | 4.9181 | 5230 | 46.4 | 66503 | 54.2 |
| 19.186 | 4.6221 | 285 | 2.5 | 2328 | 1.9 |
| 19.653 | 4.5134 | 375 | 3.3 | 3985 | 3.2 |
| 19.985 | 4.4391 | 815 | 7.2 | 7562 | 6.2 |
| 20.318 | 4.3671 | 431 | 3.8 | 3960 | 3.2 |
| 20.849 | 4.257 | 330 | 2.9 | 2227 | 1.8 |
| 21.214 | 4.1846 | 327 | 2.9 | 5080 | 4.1 |
| 21.507 | 4.1283 | 1859 | 16.5 | 19728 | 16.1 |
| 21.954 | 4.0453 | 1384 | 12.3 | 13870 | 11.3 |
| 22.752 | 3.9051 | 2219 | 19.7 | 23436 | 19.1 |
| 23.669 | 3.7559 | 165 | 1.5 | 1549 | 1.3 |
| 24.235 | 3.6695 | 654 | 5.8 | 8902 | 7.3 |
| 24.605 | 3.615 | 1655 | 14.7 | 20253 | 16.5 |
| 25.636 | 3.472 | 726 | 6.4 | 10925 | 8.9 |
| 25.989 | 3.4257 | 333 | 3 | 3446 | 2.8 |
| 26.416 | 3.3712 | 350 | 3.1 | 4065 | 3.3 |
| 26.826 | 3.3207 | 78 | 0.7 | 813 | 0.7 |
| 27.294 | 3.2647 | 229 | 2 | 5718 | 4.7 |
| 27.564 | 3.2334 | 1257 | 11.2 | 15554 | 12.7 |
| 27.996 | 3.1845 | 156 | 1.4 | 2641 | 2.2 |
| 28.556 | 3.1233 | 105 | 0.9 | 1072 | 0.9 |
| 29.277 | 3.048 | 78 | 0.7 | 808 | 0.7 |
| 29.576 | 3.0178 | 92 | 0.8 | 1239 | 1 |
| 30.153 | 2.9613 | 159 | 1.4 | 2743 | 2.2 |
| 30.663 | 2.9133 | 632 | 5.6 | 8351 | 6.8 |
| 31.578 | 2.8309 | 81 | 0.7 | 852 | 0.7 |
| 32.165 | 2.7806 | 183 | 1.6 | 2134 | 1.7 |
| 32.905 | 2.7197 | 152 | 1.3 | 2196 | 1.8 |
| 33.138 | 2.7012 | 174 | 1.5 | 1855 | 1.5 |
| 33.625 | 2.6631 | 100 | 0.9 | 1757 | 1.4 |
| 33.956 | 2.6379 | 231 | 2.1 | 4185 | 3.4 |
| 34.654 | 2.5864 | 61 | 0.5 | 745 | 0.6 |
| 35.143 | 2.5515 | 211 | 1.9 | 2564 | 2.1 |
| 35.844 | 2.5031 | 330 | 2.9 | 4242 | 3.5 |
| 36.663 | 2.4491 | 186 | 1.7 | 2753 | 2.2 |
| 37.189 | 2.4157 | 147 | 1.3 | 2384 | 1.9 |
| 37.794 | 2.3783 | 118 | 1 | 1026 | 0.8 |
| 38.786 | 2.3198 | 131 | 1.2 | 3285 | 2.7 |
| 39.469 | 2.2812 | 88 | 0.8 | 2864 | 2.3 |

The DSC curve is as shown in Figure 2B, displaying two endothermic peaks at 91.88°C and 171.38°C, respectively (onset values).

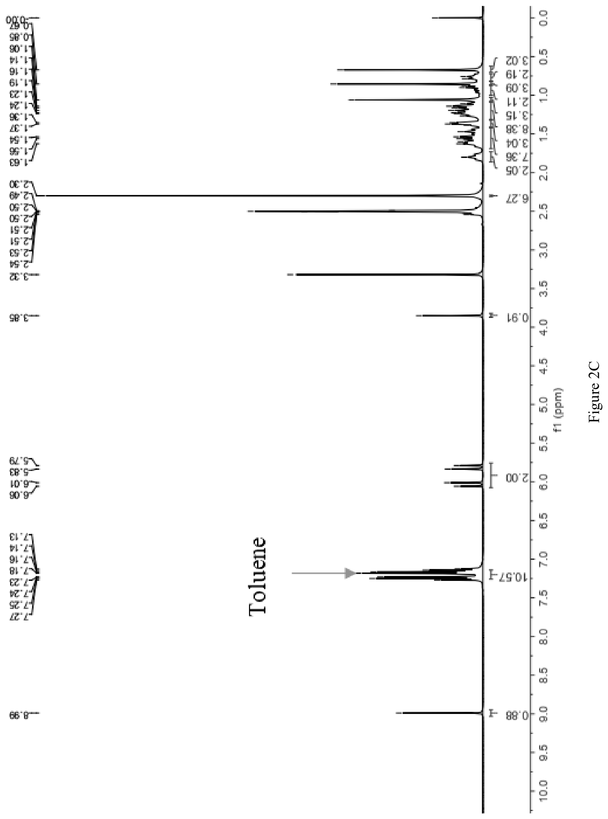

The ¹H NMR spectrum is as shown in Figure 2C. Analysis of the spectrum indicated that the sample contained approximately 30.8% residual toluene (corresponding to about 2 molecules of toluene).

### Example 3: Preparation of crystal form C

Approximately 20 mg of the compound of Formula I was added to a 3 mL vial. Then, 1 mL of methanol was added to the vial. The mixture was sonicated to promote dissolution, filtered, and the filtrate was allowed to stand for solvent evaporation.

The X-ray powder diffraction pattern is as shown in Figure 3A. The XRPD data are listed in Table 3.

**Table 3**

| Diffraction Angle [°2θ] | d value [Å] | Peak Height | I% | Area | I% |
|---|---|---|---|---|---|
| 5.579 | 15.8288 | 4134 | 38.7 | 22417 | 24.5 |
| 5.939 | 14.8702 | 153 | 1.4 | 2209 | 2.4 |
| 8.63 | 10.2379 | 514 | 4.8 | 4214 | 4.6 |
| 9.9 | 8.9269 | 340 | 3.2 | 2238 | 2.4 |
| 11.246 | 7.8617 | 381 | 3.6 | 1826 | 2 |
| 11.752 | 7.5241 | 291 | 2.7 | 5321 | 5.8 |
| 11.923 | 7.4167 | 282 | 2.6 | 5006 | 5.5 |
| 13.326 | 6.6387 | 126 | 1.2 | 896 | 1 |
| 13.947 | 6.3445 | 730 | 6.8 | 5171 | 5.6 |
| 14.555 | 6.0807 | 156 | 1.5 | 1954 | 2.1 |
| 14.886 | 5.9463 | 612 | 5.7 | 7837 | 8.5 |
| 15.697 | 5.6408 | 64 | 0.6 | 211 | 0.2 |
| 16.367 | 5.4115 | 3414 | 31.9 | 40154 | 43.8 |
| 16.673 | 5.3129 | 10689 | 100 | 91669 | 100 |
| 17.357 | 5.105 | 10137 | 94.8 | 84761 | 92.5 |
| 18.486 | 4.7956 | 3012 | 28.2 | 26569 | 29 |
| 18.859 | 4.7015 | 2043 | 19.1 | 18250 | 19.9 |
| 19.952 | 4.4465 | 475 | 4.4 | 4511 | 4.9 |
| 20.453 | 4.3387 | 213 | 2 | 5777 | 6.3 |
| 20.648 | 4.298 | 632 | 5.9 | 8121 | 8.9 |
| 21.246 | 4.1785 | 113 | 1.1 | 801 | 0.9 |
| 21.738 | 4.0849 | 1147 | 10.7 | 8380 | 9.1 |
| 22.463 | 3.9547 | 175 | 1.6 | 2605 | 2.8 |
| 22.657 | 3.9213 | 217 | 2 | 1106 | 1.2 |
| 23.32 | 3.8112 | 89 | 0.8 | 696 | 0.8 |
| 24.141 | 3.6835 | 119 | 1.1 | 1201 | 1.3 |
| 24.95 | 3.5659 | 120 | 1.1 | 814 | 0.9 |
| 25.42 | 3.501 | 900 | 8.4 | 7321 | 8 |
| 25.852 | 3.4435 | 318 | 3 | 2953 | 3.2 |
| 26.184 | 3.4006 | 159 | 1.5 | 2156 | 2.4 |
| 26.904 | 3.3111 | 283 | 2.6 | 2310 | 2.5 |
| 27.273 | 3.2672 | 117 | 1.1 | 1529 | 1.7 |
| 27.796 | 3.2069 | 166 | 1.6 | 1238 | 1.4 |
| 28.208 | 3.161 | 228 | 2.1 | 2113 | 2.3 |
| 28.634 | 3.1149 | 1026 | 9.6 | 9189 | 10 |
| 29.934 | 2.9826 | 57 | 0.5 | 258 | 0.3 |
| 30.583 | 2.9208 | 119 | 1.1 | 1534 | 1.7 |
| 31.072 | 2.8759 | 54 | 0.5 | 709 | 0.8 |
| 31.912 | 2.8021 | 84 | 0.8 | 960 | 1 |
| 32.53 | 2.7502 | 52 | 0.5 | 446 | 0.5 |
| 33.33 | 2.686 | 242 | 2.3 | 2543 | 2.8 |
| 33.822 | 2.648 | 236 | 2.2 | 2503 | 2.7 |
| 34.31 | 2.6115 | 1007 | 9.4 | 8345 | 9.1 |
| 35.434 | 2.5312 | 204 | 1.9 | 2865 | 3.1 |
| 35.907 | 2.4989 | 125 | 1.2 | 1352 | 1.5 |
| 36.51 | 2.459 | 117 | 1.1 | 1420 | 1.5 |
| 37.499 | 2.3964 | 68 | 0.6 | 883 | 1 |
| 38.337 | 2.3459 | 108 | 1 | 1037 | 1.1 |

The DSC curve is as shown in Figure 3B, displaying two endothermic peaks at 90.02°C and 174.69°C, respectively (onset values).

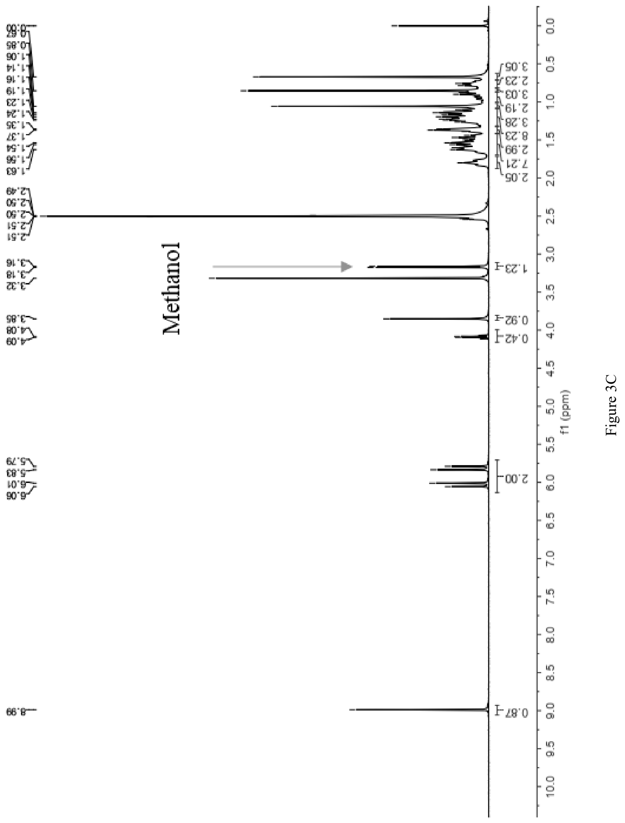

The ¹H NMR spectrum is as shown in Figure 3C. Analysis of the spectrum indicated that the sample contained approximately 3.09% residual methanol.

### Example 4: Preparation of crystal form D

Approximately 50 mg of the compound of Formula I was added to a 5 mL vial. Then, 1 mL of dichloromethane was added. The mixture was sonicated to promote dissolution, filtered, and the filtrate was subjected to rotary evaporation.

The X-ray powder diffraction pattern is as shown in Figure 4A. The XRPD data are listed in Table 4.

**Table 4**

| Diffraction Angle [°2θ] | d value [Å] | Peak Height | I% | Area | I% |
|---|---|---|---|---|---|
| 5.902 | 14.9614 | 112 | 1.3 | 648 | 0.7 |
| 7.088 | 12.4604 | 327 | 3.7 | 2176 | 2.3 |
| 7.62 | 11.5922 | 150 | 1.7 | 1147 | 1.2 |
| 8.004 | 11.037 | 194 | 2.2 | 999 | 1.1 |
| 8.76 | 10.0864 | 1631 | 18.7 | 9231 | 9.8 |
| 10.209 | 8.6574 | 356 | 4.1 | 1696 | 1.8 |
| 10.964 | 8.0627 | 172 | 2 | 1109 | 1.2 |
| 11.642 | 7.5949 | 347 | 4 | 1993 | 2.1 |
| 12.233 | 7.2292 | 912 | 10.4 | 4990 | 5.3 |
| 12.893 | 6.8606 | 593 | 6.8 | 5116 | 5.4 |
| 13.477 | 6.5648 | 1199 | 13.7 | 6874 | 7.3 |
| 13.755 | 6.4327 | 265 | 3 | 2260 | 2.4 |
| 14.047 | 6.2994 | 947 | 10.8 | 6041 | 6.4 |
| 14.419 | 6.1378 | 1637 | 18.8 | 9741 | 10.3 |
| 14.884 | 5.9472 | 205 | 2.3 | 1091 | 1.2 |
| 15.179 | 5.8321 | 395 | 4.5 | 3772 | 4 |
| 15.366 | 5.7617 | 1060 | 12.1 | 7660 | 8.1 |
| 15.875 | 5.5781 | 212 | 2.4 | 1373 | 1.5 |
| 16.093 | 5.503 | 515 | 5.9 | 3203 | 3.4 |
| 16.303 | 5.4325 | 434 | 5 | 2728 | 2.9 |
| 16.554 | 5.3508 | 485 | 5.6 | 3393 | 3.6 |
| 17.612 | 5.0315 | 8729 | 100 | 94539 | 100 |
| 18.176 | 4.8767 | 3699 | 42.4 | 31405 | 33.2 |
| 18.466 | 4.8007 | 500 | 5.7 | 12304 | 13 |
| 18.818 | 4.7118 | 227 | 2.6 | 1521 | 1.6 |
| 19.152 | 4.6302 | 166 | 1.9 | 2019 | 2.1 |
| 20.664 | 4.2948 | 312 | 3.6 | 2462 | 2.6 |
| 21.098 | 4.2074 | 170 | 1.9 | 1687 | 1.8 |
| 21.328 | 4.1625 | 444 | 5.1 | 4806 | 5.1 |
| 21.526 | 4.1247 | 727 | 8.3 | 6972 | 7.4 |
| 21.779 | 4.0773 | 135 | 1.5 | 1930 | 2 |
| 22.092 | 4.0203 | 145 | 1.7 | 1507 | 1.6 |
| 22.542 | 3.9411 | 214 | 2.5 | 2676 | 2.8 |
| 23.398 | 3.7988 | 96 | 1.1 | 1432 | 1.5 |
| 23.592 | 3.768 | 134 | 1.5 | 2903 | 3.1 |
| 23.842 | 3.7291 | 119 | 1.4 | 1778 | 1.9 |
| 24.133 | 3.6847 | 176 | 2 | 4684 | 5 |
| 24.942 | 3.567 | 72 | 0.8 | 454 | 0.5 |
| 25.265 | 3.5221 | 110 | 1.3 | 766 | 0.8 |
| 25.948 | 3.431 | 131 | 1.5 | 1287 | 1.4 |
| 26.22 | 3.396 | 215 | 2.5 | 2872 | 3 |
| 26.808 | 3.3228 | 287 | 3.3 | 2937 | 3.1 |
| 27.062 | 3.2922 | 116 | 1.3 | 1818 | 1.9 |
| 27.546 | 3.2354 | 85 | 1 | 405 | 0.4 |
| 28.405 | 3.1395 | 67 | 0.8 | 1691 | 1.8 |
| 28.697 | 3.1082 | 65 | 0.7 | 1671 | 1.8 |
| 30.081 | 2.9683 | 87 | 1 | 892 | 0.9 |
| 32.853 | 2.7239 | 36 | 0.4 | 122 | 0.1 |
| 33.376 | 2.6824 | 107 | 1.2 | 1393 | 1.5 |
| 34.229 | 2.6175 | 39 | 0.4 | 357 | 0.4 |
| 35.771 | 2.5081 | 106 | 1.2 | 1787 | 1.9 |
| 36.351 | 2.4694 | 91 | 1 | 1100 | 1.2 |
| 37.677 | 2.3855 | 114 | 1.3 | 1563 | 1.7 |
| 38.18 | 2.3552 | 110 | 1.3 | 1350 | 1.4 |
| 39.559 | 2.2762 | 87 | 1 | 1454 | 1.5 |

The DSC curve is as shown in Figure 4B, displaying two endothermic peaks at 155.68°C and 168.97°C, respectively (onset values).

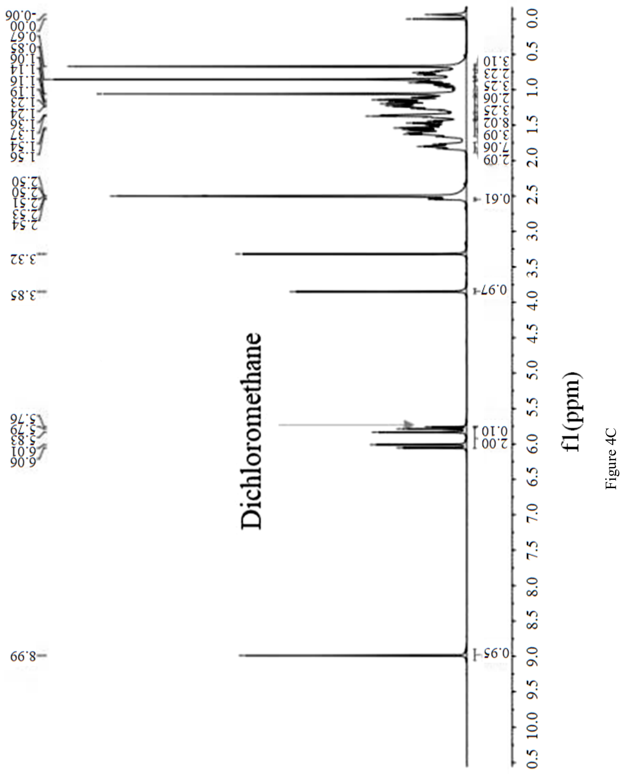

The ¹H NMR spectrum is as shown in Figure 4C. Analysis of the spectrum indicated that the sample contained approximately 1.02% residual dichloromethane.

### Example 5: Preparation of crystal form E

Approximately 20 mg of the compound of Formula I was added to a 5 mL vial. Then, 0.5 mL of methanol was added to the vial. The mixture was sonicated to promote dissolution and filtered to obtain a filtrate. The filtrate was slowly dripped into a vial pre-filled with 3 mL of water, resulting in the precipitation of solid.

The X-ray powder diffraction pattern is as shown in Figure 5A. The XRPD data are listed in Table 5.

**Table 5**

| Diffraction Angle [°2θ] | d value [Å] | Peak Height | I% | Area | I% |
|---|---|---|---|---|---|
| 6.138 | 14.388 | 150 | 14.2 | 1817 | 15.4 |
| 10.107 | 8.7442 | 47 | 4.5 | 442 | 3.7 |
| 10.649 | 8.3011 | 33 | 3.1 | 425 | 3.6 |
| 12.429 | 7.116 | 344 | 32.6 | 3333 | 28.2 |
| 13.769 | 6.4259 | 1056 | 100 | 11822 | 100 |
| 14.571 | 6.074 | 210 | 19.9 | 2166 | 18.3 |
| 15.213 | 5.8194 | 420 | 39.8 | 4520 | 38.2 |
| 15.84 | 5.5902 | 478 | 45.3 | 5086 | 43 |
| 16.387 | 5.405 | 150 | 14.2 | 1932 | 16.3 |
| 16.814 | 5.2686 | 113 | 10.7 | 1451 | 12.3 |
| 17.589 | 5.0382 | 308 | 29.2 | 4515 | 38.2 |
| 17.863 | 4.9614 | 628 | 59.5 | 7664 | 64.8 |
| 18.236 | 4.8608 | 98 | 9.3 | 2665 | 22.5 |
| 18.722 | 4.7358 | 460 | 43.6 | 4717 | 39.9 |
| 20.047 | 4.4255 | 134 | 12.7 | 2583 | 21.8 |
| 20.335 | 4.3636 | 141 | 13.4 | 2064 | 17.5 |
| 21.313 | 4.1655 | 262 | 24.8 | 5914 | 50 |
| 21.64 | 4.1032 | 177 | 16.8 | 4269 | 36.1 |
| 21.974 | 4.0416 | 67 | 6.3 | 1111 | 9.4 |
| 22.91 | 3.8785 | 211 | 20 | 2967 | 25.1 |
| 23.96 | 3.7109 | 49 | 4.6 | 1013 | 8.6 |
| 24.484 | 3.6326 | 70 | 6.6 | 1232 | 10.4 |
| 25.032 | 3.5544 | 52 | 4.9 | 431 | 3.6 |
| 25.421 | 3.5009 | 59 | 5.6 | 793 | 6.7 |
| 26.007 | 3.4233 | 76 | 7.2 | 1377 | 11.6 |
| 27 | 3.2996 | 213 | 20.2 | 3510 | 29.7 |
| 28.247 | 3.1567 | 73 | 6.9 | 1738 | 14.7 |
| 29.43 | 3.0324 | 50 | 4.7 | 644 | 5.4 |
| 30.16 | 2.9607 | 34 | 3.2 | 441 | 3.7 |
| 30.782 | 2.9023 | 79 | 7.5 | 1212 | 10.3 |
| 31.636 | 2.8258 | 128 | 12.1 | 2201 | 18.6 |
| 32.842 | 2.7248 | 42 | 4 | 918 | 7.8 |
| 33.194 | 2.6967 | 42 | 4 | 918 | 7.8 |
| 33.776 | 2.6516 | 45 | 4.3 | 629 | 5.3 |
| 36.198 | 2.4795 | 107 | 10.1 | 2144 | 18.1 |
| 39.017 | 2.3066 | 48 | 4.5 | 990 | 8.4 |
| 39.585 | 2.2748 | 175 | 16.6 | 3249 | 27.5 |

The DSC curve is as shown in Figure 5B, displaying a single endothermic peak with a melting point at 170.16°C (onset value).

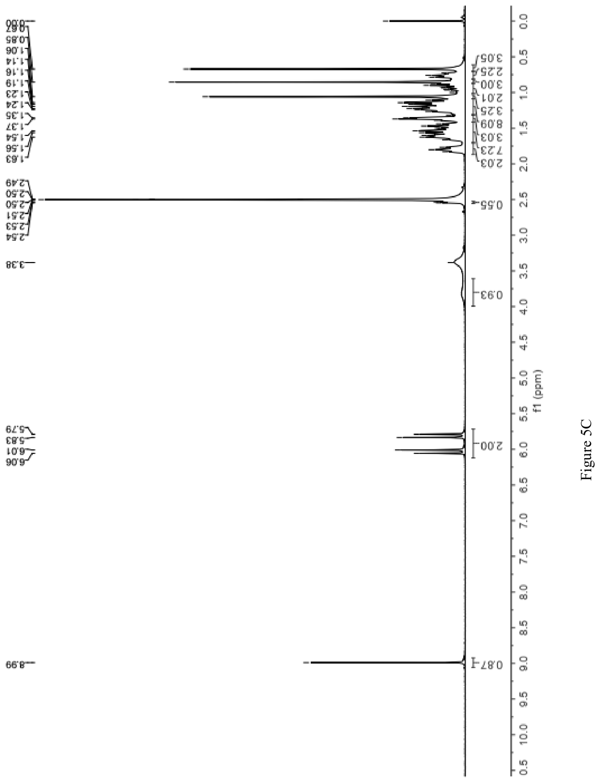

The ¹H NMR spectrum is as shown in Figure 5C. Analysis of the spectrum indicated no significant solvent residue in the sample.

### Example 6: Preparation of crystal form F

Preparation method: Approximately 200 mg of the compound of Formula I was added to a 20 mL vial. Then, 4 mL of methanol was added to the vial, and a stir bar was added. The mixture was stirred at room temperature overnight (for approximately 18 hours), then filtered. The resulting solid was dried at 50°C overnight (for approximately 18 hours).

The X-ray powder diffraction pattern is as shown in Figure 6A. The XRPD data are listed in Table 6.

**Table 6**

| Diffraction Angle [°2θ] | d value [Å] | Peak Height | I% | Area | I% |
|---|---|---|---|---|---|
| 5.96 | 14.8166 | 157 | 1.3 | 857 | 0.7 |
| 8.688 | 10.1699 | 946 | 7.8 | 7648 | 6.2 |
| 11.983 | 7.3795 | 346 | 2.8 | 3000 | 2.4 |
| 12.484 | 7.0846 | 230 | 1.9 | 1671 | 1.3 |
| 13.522 | 6.5427 | 315 | 2.6 | 2745 | 2.2 |
| 13.949 | 6.3437 | 44 | 0.4 | 639 | 0.5 |
| 14.629 | 6.0502 | 500 | 4.1 | 7339 | 5.9 |
| 14.943 | 5.9237 | 4147 | 34.2 | 32851 | 26.5 |
| 15.838 | 5.591 | 241 | 2 | 1205 | 1 |
| 16.402 | 5.4 | 9346 | 77 | 97155 | 78.4 |
| 16.658 | 5.3176 | 4785 | 39.4 | 66818 | 53.9 |
| 17.474 | 5.0709 | 12143 | 100 | 123884 | 100 |
| 18.473 | 4.799 | 275 | 2.3 | 5442 | 4.4 |
| 18.741 | 4.731 | 1178 | 9.7 | 14969 | 12.1 |
| 18.972 | 4.6738 | 230 | 1.9 | 6519 | 5.3 |
| 19.36 | 4.581 | 216 | 1.8 | 1269 | 1 |
| 19.849 | 4.4693 | 242 | 2 | 1922 | 1.6 |
| 20.415 | 4.3465 | 1368 | 11.3 | 21531 | 17.4 |
| 20.687 | 4.2901 | 2102 | 17.3 | 20334 | 16.4 |
| 21.059 | 4.2152 | 137 | 1.1 | 2616 | 2.1 |
| 21.302 | 4.1676 | 54 | 0.4 | 280 | 0.2 |
| 21.721 | 4.0881 | 45 | 0.4 | 420 | 0.3 |
| 22.343 | 3.9756 | 503 | 4.1 | 5151 | 4.2 |
| 23.355 | 3.8056 | 389 | 3.2 | 3242 | 2.6 |
| 23.789 | 3.7373 | 245 | 2 | 2656 | 2.1 |
| 24.171 | 3.679 | 60 | 0.5 | 642 | 0.5 |
| 24.395 | 3.6457 | 62 | 0.5 | 422 | 0.3 |
| 24.917 | 3.5705 | 161 | 1.3 | 1565 | 1.3 |
| 25.478 | 3.4931 | 347 | 2.9 | 4798 | 3.9 |
| 25.811 | 3.4489 | 170 | 1.4 | 1690 | 1.4 |
| 26.36 | 3.3782 | 293 | 2.4 | 2995 | 2.4 |
| 27.643 | 3.2243 | 808 | 6.7 | 14248 | 11.5 |
| 29.067 | 3.0696 | 258 | 2.1 | 4119 | 3.3 |
| 31.618 | 2.8274 | 93 | 0.8 | 1154 | 0.9 |
| 33.271 | 2.6906 | 278 | 2.3 | 7006 | 5.7 |
| 33.801 | 2.6497 | 71 | 0.6 | 1447 | 1.2 |
| 34.924 | 2.567 | 48 | 0.4 | 918 | 0.7 |
| 35.629 | 2.5178 | 348 | 2.9 | 5082 | 4.1 |
| 36.054 | 2.4891 | 86 | 0.7 | 1759 | 1.4 |
| 36.7 | 2.4467 | 111 | 0.9 | 2117 | 1.7 |
| 39.682 | 2.2694 | 73 | 0.6 | 760 | 0.6 |

The DSC curve is as shown in Figure 6B, displaying two endothermic peaks at 83.04°C and 177.93°C, respectively (onset values).

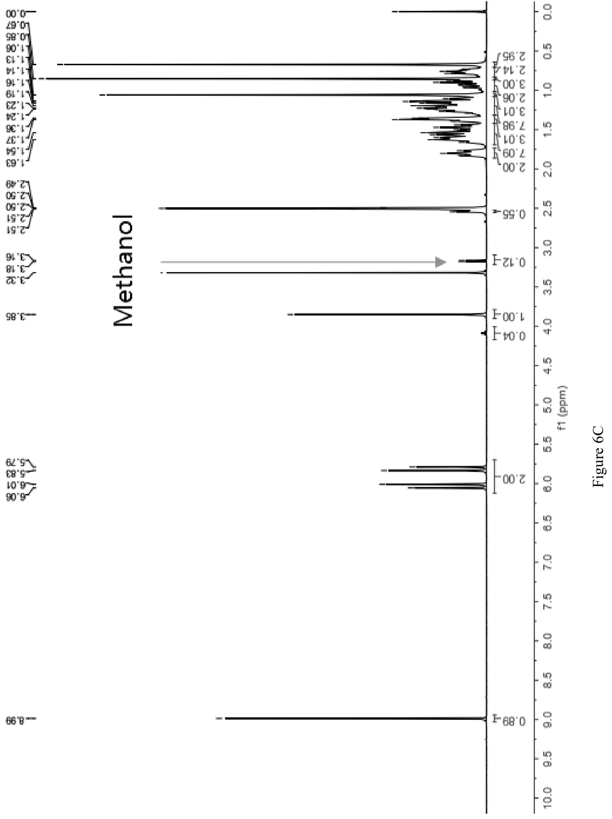

The ¹H NMR spectrum is as shown in Figure 6C. Analysis of the spectrum indicated that the sample contained approximately 0.31% residual methanol.

### Example 7: Preparation of crystal form G

Preparation method: Crystal form F compound was placed on a hot stage, heated to 125°C and maintained for approximately 10 minutes, resulting in the conversion of crystal form F to crystal form G.

The X-ray powder diffraction pattern is as shown in Figure 7A. The XRPD data are listed in Table 7.

**Table 7**

| Diffraction Angle [°2θ] | d value [Å] | Peak Height | I% | Area | I% |
|---|---|---|---|---|---|
| 6.158 | 14.3404 | 67 | 1.5 | 268 | 0.6 |
| 8.355 | 10.5739 | 327 | 7.1 | 3599 | 8 |
| 8.728 | 10.1225 | 59 | 1.3 | 682 | 1.5 |
| 10.075 | 8.7726 | 354 | 7.7 | 3039 | 6.8 |
| 10.717 | 8.2484 | 94 | 2.1 | 781 | 1.7 |
| 12.427 | 7.117 | 69 | 1.5 | 722 | 1.6 |
| 13.714 | 6.4516 | 1083 | 23.6 | 12654 | 28.3 |
| 14.551 | 6.0824 | 231 | 5 | 2068 | 4.6 |
| 15.194 | 5.8264 | 1018 | 22.2 | 8404 | 18.8 |
| 15.837 | 5.5913 | 1303 | 28.4 | 11649 | 26 |
| 16.402 | 5.4 | 152 | 3.3 | 862 | 1.9 |
| 16.811 | 5.2696 | 2273 | 49.6 | 23310 | 52.1 |
| 17.551 | 5.0488 | 4581 | 100 | 44776 | 100 |
| 18.294 | 4.8456 | 387 | 8.4 | 4357 | 9.7 |
| 18.718 | 4.7366 | 164 | 3.6 | 416 | 0.9 |
| 19.425 | 4.5657 | 318 | 6.9 | 3283 | 7.3 |
| 20.32 | 4.3668 | 578 | 12.6 | 8455 | 18.9 |
| 21.312 | 4.1656 | 146 | 3.2 | 3657 | 8.2 |
| 21.606 | 4.1097 | 458 | 10 | 9261 | 20.7 |
| 21.918 | 4.0519 | 310 | 6.8 | 5719 | 12.8 |
| 22.89 | 3.882 | 125 | 2.7 | 3196 | 7.1 |
| 23.202 | 3.8304 | 146 | 3.2 | 3196 | 7.1 |
| 23.979 | 3.7081 | 110 | 2.4 | 2244 | 5 |
| 24.447 | 3.6381 | 78 | 1.7 | 1858 | 4.1 |
| 24.984 | 3.5611 | 38 | 0.8 | 177 | 0.4 |
| 25.371 | 3.5077 | 67 | 1.5 | 904 | 2 |
| 25.735 | 3.4589 | 261 | 5.7 | 4553 | 10.2 |
| 26.136 | 3.4068 | 101 | 2.2 | 1873 | 4.2 |
| 27.015 | 3.2978 | 61 | 1.3 | 1438 | 3.2 |
| 29.339 | 3.0416 | 183 | 4 | 3580 | 8 |
| 30.742 | 2.906 | 77 | 1.7 | 918 | 2.1 |
| 31.598 | 2.8292 | 48 | 1 | 719 | 1.6 |
| 35.629 | 2.5177 | 60 | 1.3 | 1063 | 2.4 |
| 36.263 | 2.4752 | 32 | 0.7 | 235 | 0.5 |
| 39.62 | 2.2729 | 43 | 0.9 | 616 | 1.4 |

The DSC curve is as shown in Figure 7B, displaying a single endothermic peak with a melting point at 177.75°C (onset value).

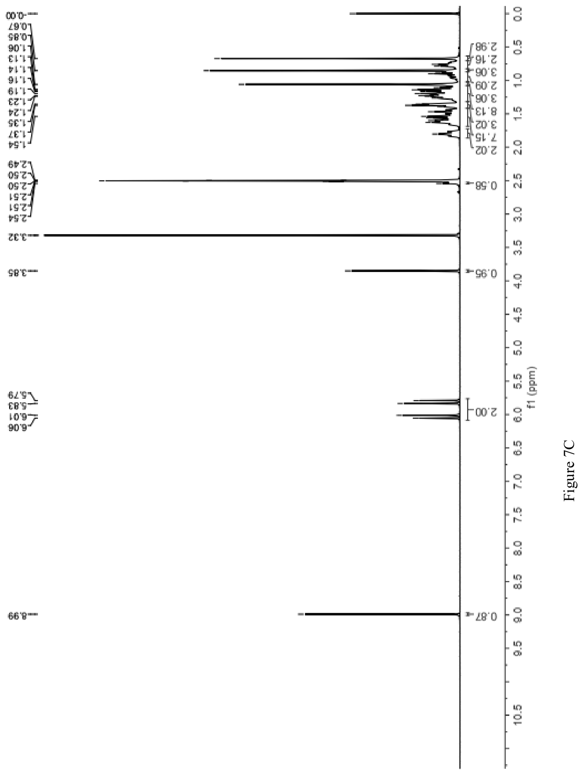

The ¹H NMR spectrum is as shown in Figure 7C. Analysis of the spectrum indicated no significant solvent residue in the sample.

### Example 8: Preparation of crystal form H

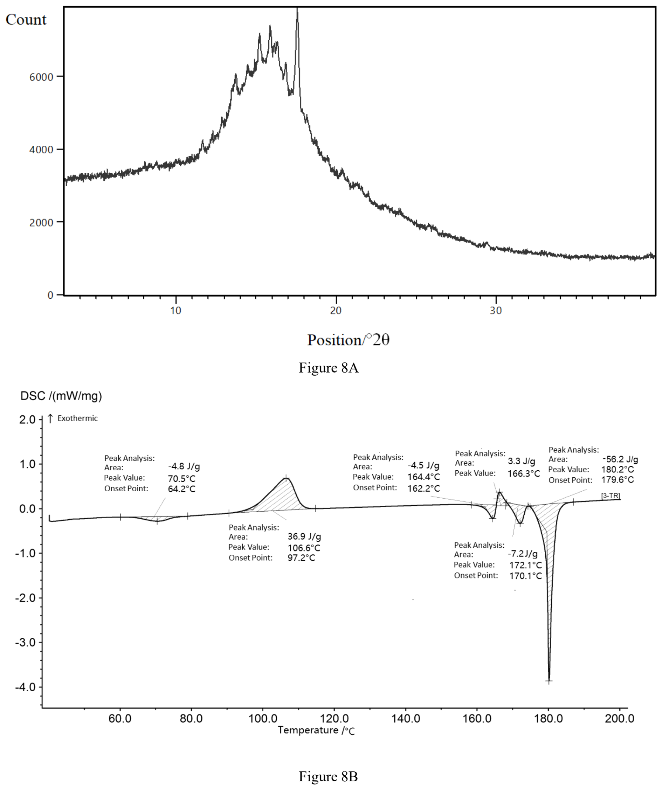

Preparation method: Following the method described in Example 20 of WO2020143640A1, the KHC-8 compound was reacted with acetonitrile, cesium carbonate, and 1H-tetrazole. Subsequently, water was added, and the mixture was extracted with ethyl acetate. The organic phases were combined and washed with saturated sodium chloride solution, dried over anhydrous sodium sulfate, and the solvent was removed under reduced pressure to obtain the crude compound of Formula I.
1) 1.370 g of the crude compound of Formula I was dissolved in 31 mL of a mixed solvent (Dichloromethane (DCM)/Methanol (MeOH) = 30/1). The solution was applied onto a preparative TLC plate. The plate was then developed using DCM/MeOH (30/1) as the eluate (Rf = 0.5). After the plate was completely dried, the solid compound on the plate was scraped off and subjected to XRPD analysis. The X-ray powder diffraction pattern is as shown in Figure 8A.
2) 1.372 g of the crude compound of Formula I was eluted using 250 mL of a mixed solvent (DCM/MeOH = 30/1). The eluate was collected and concentrated under reduced pressure at 45°C to yield a white solid. The X-ray powder diffraction pattern was substantially consistent with Figure 8A.

The solid finally obtained from step 1) or 2) was subjected to DSC testing. The DSC curve is as shown in Figure 8B. Analysis of the DSC curve revealed the following: a small endothermic peak near 64°C, which may correspond to desolvation; an exothermic peak near 97°C, which is correspond to the recrystallization of amorphous form; and three endothermic peaks at approximately 162°C, 170°C, and 179°C, indicating that the obtained solid is a mixture of crystal forms.

### Example 9: Interconversion experiments of anhydrous crystal forms

To clarify the stability relationships among the anhydrous crystal forms Form A, Form E and Form G under different temperature conditions, suspension competition experiments were conducted in various solvents at 25°C and 50°C. The specific procedure was as follows: First, saturated solutions of the starting material, compound of Formula I, were prepared in the corresponding solvents at the specified temperatures. Equal weights of Form A and Form E were separately added to the filtered saturated solutions to form suspension. The suspension was stirred at 25°C and 50°C, respectively, for 4 days, followed by sampling for XRPD analysis. Equal weights of Form A and Form G were separately added to the filtered saturated solutions to form suspension, which were stirred at 25°C and 50°C, respectively, for one day, samples were taken for XRPD analysis. The results are shown in Table 9.

**Table 9**

| Solid-state form of starting material | Solvent | Temperature (°C) | Experimental Results |
|---|---|---|---|
| Form A/E | Methyl tert-butyl ether | 25 | Form A |
| Form A/E | Acetonitrile | 25 | Form A |
| Form A/E | Methyl tert-butyl ether | 50 | Form A |
| Form A/E | Acetonitrile | 50 | Form A |
| Form A/G | Acetonitrile | 25 | Form A |
| Form A/G | Acetonitrile | 50 | Form A |

### Example 10: Grinding stability experiment

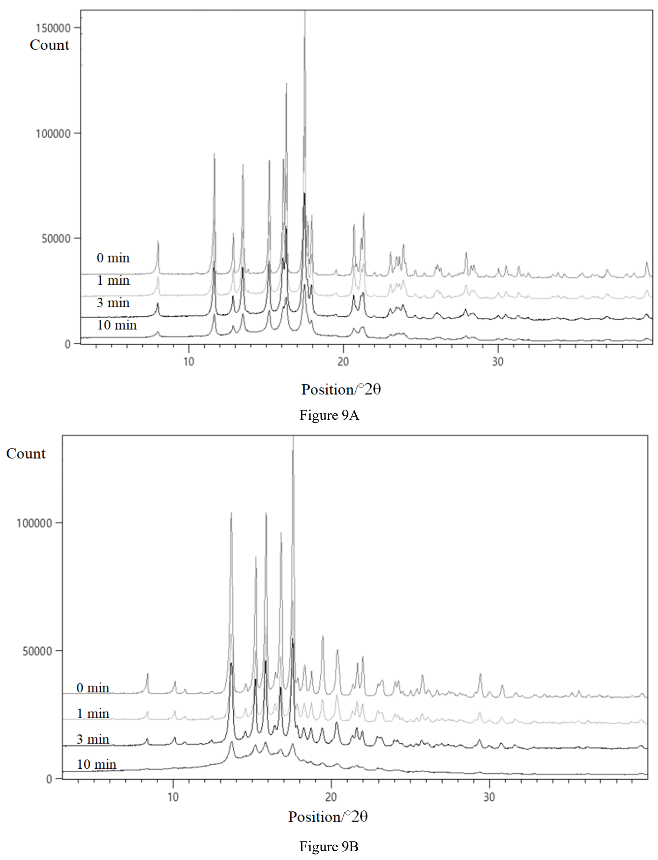

Crystal form A and crystal form G were ground separately for 1 minute, 3 minutes, and 10 minutes. Their crystal form changes were then examined by PXRD. The results are shown in Figure 9A and Figure 9B. The results indicate that after 10 minutes of grinding, crystal form A remained unchanged, whereas crystal form G became semi-amorphous. This demonstrates that crystal form A possesses better stability under grinding conditions compared to crystal form G.

### Example 11: Storage stability experiment

Appropriate amounts of test samples were placed in open containers and subjected to the respective influencing factors (high temperature, high humidity, light exposure). Samples were taken at 0 days, 5 days, 12 days, and 30 days for crystal form analysis and related substance content testing. Specific experimental conditions for the influencing factors are detailed in the table below. Note: Constant humidity conditions were achieved using saturated salt solutions placed at the bottom of desiccators, with 92.5% RH achieved by a saturated KNO₃ solution.

**Table 10: Experimental condition parameters for influencing factors**

| Influencing factors | Experimental condition parameters |
|---|---|
| High temperature | 60°C±2°C |
| High humidity | 25°C±5°C, 92.5%±5%RH |
| Light exposure | 4500lx±500lx |

The test results indicated that after 30 days of storage under high temperature, high humidity, and light exposure conditions, the maximum unknown individual impurity and total impurities for crystal form A and crystal form G showed no significant increase compared to day 0. However, for crystal form H, the total impurities increased by 0.9% relative to day 0 after 30 days of light exposure. Crystal form A and crystal form G remained relatively stable in their crystal form after 30 days of storage under high temperature, high humidity, and light exposure conditions. However, crystal form H underwent a solid-state form transformation after only 5 days under high temperature, high humidity, and light exposure condition, primarily converting into a mixture of crystal form A with a minor amount of crystal form G.

## Claims

1. A crystal form A of a compound of Formula I, wherein: 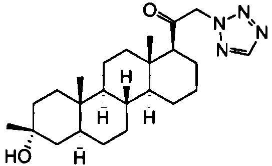 the X-ray powder diffraction pattern thereof has characteristic peaks at 2θ values of 8.1°±0.2°, 11.7°±0.2°, 13.5°±0.2°, 16.2°±0.2°, and 17.6°±0.2°; preferably, the X-ray powder diffraction pattern of the crystal form A has characteristic peaks at 2θ values of 8.1°±0.2°, 11.7°±0.2°, 13.0°+0.2°, 13.5°±0.2°, 15.3°±0.2°, 16.2°±0.2°, 17.6°±0.2°, and 21.3°±0.2°; more preferably, the X-ray powder diffraction pattern of the crystal form A is substantially consistent with Figure 1A.

2. A crystal form B of a compound of Formula I, wherein the X-ray powder diffraction pattern thereof has characteristic peaks at 2θ values of 7.3°+0.2°, 9.0°+0.2°, 14.7°±0.2°, 15.6°±0.2°, and 18.0°±0.2°; preferably, the X-ray powder diffraction pattern of the crystal form B has characteristic peaks at 2θ values of 7.3°±0.2°, 9.0°±0.2°,11.0°±0.2°, 12.2°±0.2°, 14.7°±0.2°, 15.3°±0.2°, 15.6°±0.2°, and 18.0°±0.2°; more preferably, the X-ray powder diffraction pattern of the crystal form B is substantially consistent with Figure 2A.

3. A crystal form C of a compound of Formula I, wherein the X-ray powder diffraction pattern thereof has characteristic peaks at 2θ values of 5.6°±0.2°, 8.6°±0.2°, 14.0°±0.2°, 16.7°±0.2°, and 17.4°±0.2°; preferably, the X-ray powder diffraction pattern of the crystal form C has characteristic peaks at 2θ values of 5.6°±0.2°, 8.6°±0.2°, 9.9°±0.2°, 14.0°±0.2°, 14.9°±0.2°, 16.7°±0.2°, 17.4°±0.2°, and 18.5°±0.2°; preferably, the X-ray powder diffraction pattern of the crystal form C has characteristic peaks at 2θ values of 5.6°±0.2°, 8.6°±0.2°, 9.9°±0.2°, 11.2°±0.2°, 11.8°±0.2°, 14.0°±0.2°, 14.9°±0.2°, 16.7°±0.2°, 17.4°±0.2°, 18.5°±0.2°, and 18.9°+0.2°; more preferably, the X-ray powder diffraction pattern of the crystal form C is substantially consistent with Figure 3A.

4. A crystal form D of a compound of Formula I, wherein the X-ray powder diffraction pattern thereof has characteristic peaks at 2θ values of 7.1°±0.2°, 8.8°±0.2°, 12.2°±0.2°, 14.4°±0.2°, and 17.6°±0.2°; preferably, the X-ray powder diffraction pattern of the crystal form D has characteristic peaks at 2θ values of 7.1°+0.2°, 8.8°±0.2°, 10.2°±0.2°, 12.2°±0.2°, 12.9°+0.2°, 13.5°+0.2°, 14.4°±0.2°, and 17.6°±0.2°; preferably, the X-ray powder diffraction pattern of the crystal form D has characteristic peaks at 2θ values of 7.1°+0.2°, 7.6°+0.2°, 8.0°±0.2°, 8.8°+0.2°, 10.2°±0.2°, 11.0°±0.2°, 11.6°+0.2°, 12.2°±0.2°, 12.9°±0.2°, 13.5°±0.2°, 14.0°±0.2°, 14.4°±0.2°, 15.4°±0.2°, 17.6°±0.2°, and 18.2°+0.2°; more preferably, the X-ray powder diffraction pattern of the crystal form D is substantially consistent with Figure 4A.

5. A crystal form E of a compound of Formula I, wherein the X-ray powder diffraction pattern thereof has characteristic peaks at 2θ values of 6.1°±0.2°, 12.4°±0.2°, 13.8°+0.2°, 15.8°+0.2°, and 17.9°±0.2°; preferably, the X-ray powder diffraction pattern of the crystal form E has characteristic peaks at 2θ values of 6.1°+0.2°, 12.4°±0.2°, 13.8°±0.2°, 14.6°±0.2°, 15.2°+0.2°, 15.8°+0.2°, 17.9°±0.2°, and 18.7°±0.2°; more preferably, the X-ray powder diffraction pattern of the crystal form E has characteristic peaks at 2θ values of 6.1°+0.2°, 10.1°±0.2°, 12.4°±0.2°, 13.8°±0.2°, 14.6°+0.2°, 15.2°+0.2°, 15.8°±0.2°, 17.6°+0.2°, 17.9°±0.2°, 18.7°±0.2°, 20.0°±0.2°, 21.6°±0.2°, and 22.9°±0.2°; more preferably, the X-ray powder diffraction pattern of the crystal form E is substantially consistent with Figure 5A.

6. A crystal form F of a compound of Formula I, wherein the X-ray powder diffraction pattern thereof has characteristic peaks at 2θ values of 8.7°±0.2°, 14.9°±0.2°, 16.4°+0.2°, 17.5°+0.2°, and 20.4°±0.2°; preferably, the X-ray powder diffraction pattern of the crystal form F has characteristic peaks at 2θ values of 8.7°+0.2°, 12.0°±0.2°, 14.6°+0.2°, 14.9°±0.2°, 16.4°+0.2°, 17.5°±0.2°, 18.7°±0.2°, and 20.4°±0.2°; more preferably, the X-ray powder diffraction pattern of the crystal form F is substantially consistent with Figure 6A.

7. A crystal form G of a compound of Formula I, wherein the X-ray powder diffraction pattern thereof has characteristic peaks at 2θ values of 8.4°+0.2°, 10.1°±0.2°, 13.7°±0.2°, 16.8°+0.2°, and 17.6°±0.2°; preferably, the X-ray powder diffraction pattern of the crystal form G has characteristic peaks at 2θ values of 8.4°+0.2°, 10.1°±0.2°, 13.7°±0.2°, 14.6°±0.2°, 15.2°+0.2°, 15.8°+0.2°, 16.8°±0.2°, and 17.6°±0.2°; more preferably, the X-ray powder diffraction pattern of the crystal form G is substantially consistent with Figure 7A.

8. A method for preparing the crystal form A according to claim 1, wherein the method comprises: adding the compound of Formula I into an organic solvent to achieve complete dissolution, then lowering the temperature to induce precipitation, and obtaining a solid after centrifugation.

9. The method according to claim 8, wherein the organic solvent is isopropanol, ethyl propyl acetate or acetonitrile, preferably isopropanol.

10. The method according to claim 8 or 9, wherein a mass-to-volume ratio of the compound of Formula I to the organic solvent is from 100:1 to 5:1, preferably from 50:1 to 10:1, more preferably 20:1.

11. A pharmaceutical composition, wherein the pharmaceutical composition comprises the crystal form according to any one of claims 1-7, and a pharmaceutically acceptable excipient.

12. Use of the crystal form according to any one of claims 1-7 in the preparation of a medicament for a disease related to a GABAA receptor.

13. The use according to claim 12, wherein the disease related to the GABAA receptor is a nervous system disease or a metabolic disease.

14. The use according to claim 12, wherein the nervous system disease is selected from the group consisting of: sleep disorders, mood disorders, schizophrenia spectrum disorders, spastic disorders, memory disorders and/or cognitive disorders, movement disorders, personality disorders, autism spectrum disorders, pain, traumatic brain injury, vascular diseases, substance abuse disorders and/or withdrawal syndromes, and tinnitus; preferably, the mood disorder is depression; wherein the depression comprises disruptive mood dysregulation disorder, major depressive disorder, persistent depressive disorder, premenstrual dysphoric disorder, substance/medication-induced disorder, disorders due to other physical disease, other specified depressive disorder and unspecified depressive disorder; more preferably, said depression is selected from the group consisting of mild depression, moderate depression, major depression, and postpartum depression; more preferably, the nervous system disease is major depression or postpartum depression.

15. The use according to claim 12, wherein the metabolic disease is selected from the group consisting of obesity, hyperlipidemia and hypertension.
